Europäisches Patentamt

(19) European Patent Office

Office européen des brevets

(11) Numéro de publication: **0 194 187**
**B1**

(12) **FASCICULE DE BREVET EUROPEEN**

(45) Date de publication du fascicule du brevet: **03.10.90**

(51) Int. Cl.⁵: **A 61 B 17/60**

(21) Numéro de dépôt: **86400350.4**

(22) Date de dépôt: **19.02.86**

(54) **Dispositif de fixateur externe à usage orthopédique.**

(30) Priorité: **22.02.85 FR 8502577**

(43) Date de publication de la demande:
**10.09.86 Bulletin 86/37**

(45) Mention de la délivrance du brevet:
**03.10.90 Bulletin 90/40**

(84) Etats contractants désignés:
**BE DE GB IT SE**

(56) Documents cités:
**EP-A-0 029 298**
**EP-A-0 104 044**
**BE-A- 641 430**
**DE-A- 867 429**
**DE-A-3 345 276**
**FR-A-2 338 692**
**US-A-2 055 024**
**US-A-4 483 334**

(73) Titulaire: **Hardy, Jean-Marie**
**Chateau de Noailles**
**F-19600 Larches (FR)**

(72) Inventeur: **Hardy, Jean-Marie**
**Chateau de Noailles**
**F-19600 Larches (FR)**

(74) Mandataire: **Hirsch, Marc-Roger**
**Cabinet Hirsch 34 rue de Bassano**
**F-75008 Paris (FR)**

Courier Press, Leamington Spa, England.

**Description**

L'invention a pour objet un dispositif de fixateur externe à usage orthopédique et vise, plus particulièrement, un tel dispositif propre à être utilisé pour la mise en oeuvre de la méthode dite d'ILIZAROV.

Dans celle-ci, comme connu, on associe à un os et/ou fragment d'os un couple d'aiguilles ou broches de petit diamètre qui se croisent sensiblement à angle droit dans chaque structure osseuse, respectivement, et après que les extrémités des aiguilles aient été assujetties à des supports extérieurs aux membres traités, lesdites aiguilles sont mises sous tension et les supports auxquels sont fixées les aiguilles sont reliés entre eux par des entretoises de longueur réglable qui comportent des moyens de mise en compression ou en extension de l'ensemble du système pour l'application d'une ou d'action(s) dynamique(s) auxdits os ou fragments d'os.

Ces entretoises sont fixées en position prédéterminée grâce à la disposition de trous ou perforations à distance constante les uns des autres sur les supports précités.

On connaît déjà des fixateurs externes prévus pour la mise en oeuvre de la méthode d'ILIZAROV, c'est-à-dire des appareils utilisés aussi bien pour le traitement par compression-extension de fractures, que pour le traitement de cals vicieux des os tubulaires longs, ou encore des traitements d'élongations pouvant être opérés sans effusion de sang, etc.

Ces appareils connus comprennent des anneaux ou demi-anneaux en profilé métallique plat comprenant des trous prépositionnés pour la fixation desdites entretoises et qui sont propres à être reliés entre eux par des tiges métalliques, pleines, de mise sous tension ou extension, à vis, écrous et contre-écrous. L'ensemble est lourd et malaisé à manipuler en raison du nombre important de pièces de sorte que l'appareil est relativement mal adapté à son utilisation dans un milieu médical moderne.

FR-A-2 338 692 décrit un fixateur externe pour la réduction des fractures osseuses, comportant des fiches de transfixion des segments osseux, des éléments d'assemblage permettant de rendre solidaires entre elles au moins deux fiches de transfixion et des organes de blocage permettant la mise en place des groupes de fiches les uns par rapport aux autres. A chaque groupe de fiches est lié un arceau articulé et les arceaux articulés sont liés deux par deux par au moins trois barres de liaison de manière à en assurer l'immobilisation.

Dans le même domaine de réalisation EP-A-0 029 298 concerne un dispositif de fixation externe pour la réduction des fractures et qui comporte des segments de cadre arqués formés de deux demi-arceaux reliés par deux tiges réglables et portant des broches de transfixion de l'os.

Ces appareils connus ne sont pas démunis d'inconvénients sur le plan médico-chirurgical. En effet, la structure même des anneaux ou demi-anneaux en profilé métallique et la position définie des supports de broches se trouvant sur ces anneaux ou demi-anneaux ne permettent pas d'obtenir le positionnement précis qui est souhaité par les praticiens, notamment sur le plan angulaire, de sorte qu'avec les appareils connus, la méthode dite d'ILIZAROV n'a pas été utilisée autant qu'elle aurait pu l'être.

C'est, d'une façon générale, un but de l'invention de proposer un dispositif de fixateur externe à usage orthopédique propre à être utilisé dans la méthode ILIZAROV, et qui soit de mise en oeuvre plus simple que les dispositifs connus.

C'est également un but de l'invention de proposer un tel dispositif à nombre d'éléments réduit, qui soit ainsi facile à transporter et qui, de ce fait, puisse trouver application dans des conditions d'intervention chirurgicale difficiles.

C'est aussi un but de l'invention de proposer un tel dispositif qui supprime les inconvénients rapportés ci-dessus des dispositifs connus eu égard notamment au positionnement précis de ses parties constitutives par rapport aux os à traiter.

C'est encore un but de l'invention de proposer un tel dispositif qui accroisse de manière considérable les possibilités de la thérapeutique chirurgicale en raison, notamment, de l'adaptabilité de l'appareil selon l'invention aux différents cas d'espèce, mais aussi en raison de sa facilité de pose et de dépose ainsi que de ses possibilités de réglage.

Un dispositif de fixateur externe à usage orthopédique propre à être utilisé pour la mise en oeuvre de la méthode dite d'ILIZAROV, comprenant au moins deux anneaux pour la fixation sensiblement en croix de broches ou aiguilles, des entretoises étant interposées entre lesdits anneaux et comportant des moyens de réglage de leur longueur pour la mise en traction ou en compression d'une structure osseuse à laquelle sont reliés, respectivement, lesdits anneaux par lesdites broches ou aiguilles, des moyens étant en outre prévus pour la mise sous tension desdites broches ou aiguilles, est caractérisé, selon l'invention, en ce que les anneaux (1, 4) sont formés d'un tube de section circulaire sur lequel sont fixés et immobilisés en position des colliers articulés (7, 8, 9—7', 8', 9') reliés respectivement, après positionnement et blocage en position angulaire aux aiguilles (10, 11—10', 11') et aux entretoises (12), les colliers (7, 7', 8, 8') reliés aux aiguilles (10, 11, 10', 11') recevant de façon orientable et amovible des organes (60) comportant des moyens (94) de mise en traction réglable desdites aiguilles (10, 11—10', 11') tandis que les colliers (9, 9') reliés aux entretoises comportent des moyens d'assemblages à articulation (14, 29, 30).

Suivant une formé de réalisation de l'invention, les organes de fixation et de mise en traction réglable des broches ou aiguilles comprennent un fourreau taraudé propre à être introduit dans l'oeil d'une pièce coopérant avec un collier articulé et un équipage comportant un anneau de fixation de l'extrémité de la broche ou aiguille auquel est associé en translation un manchon solidaire d'un écrou prolongé par une tige filetée, qui coopère avec le fourreau, ledit manchon étant monté à rotation par rapport à l'anneau au moyen de billes ou analogues.

Suivant une forme de réalisation de l'invention, à la place de l'équipage peut être préalablement monté un dispositif de forage.

Suivant une forme de réalisation de l'invention, les anneaux sont formés de deux demi-arceaux par des articulations du type à rotule crantée et les entretoises de longueur réglable sont réglable sont reliées auxdits anneaux au moyen d'attaches à deux colliers articulés.

L'invention sera bien comprise par la description qui suit, faite à titre d'exemple et en référence au dessin annexés dans lequel:

la figure 1 est une vue en perspective d'un dispositif selon l'invention dans sa condition d'utilisation, par exemple pour le traitement d'une fracture d'un os long;

la figure 2 est une vue en élévation d'une rotule d'un dispositif selon l'invention;

la figure 3 est une vue en perspective, éclatée, de ladite rotule;

la figure 4 est une vue en coupe selon la ligne IV—IV de la figure 1;

la figure 5 est une vue en élévation d'une attache propre à équiper un dispositif selon l'invention;

la figure 5a est une vue schématique, partielle et en perspective d'un dispositif selon l'invention équipé d'une attache selon la figure 5;

la figure 6 est une vue prise selon la ligne VI—VI de la figure 1;

la figure 7 est une vue en coupe selon la ligne VII—VII de la figure 6;

la figure 8 est une vue en coupe d'un collier d'un dispositif selon l'invention;

la figure 9 est une vue analogue à la figure 5, mais pour une autre forme de réalisation;

la figure 10 est une vue, à plus grande échelle, d'un moyen de mise sous tension d'une broche d'un dispositif selon l'invention;

la figure 11 est une vue schématique, en perspective, d'un dispositif selon l'invention pour une autre forme d'utilisation.

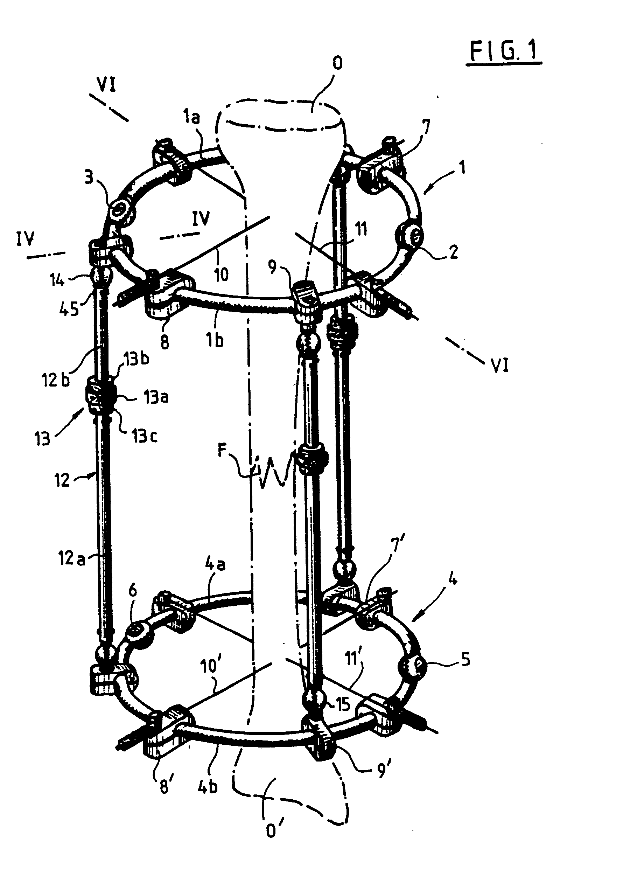

On se réfère d'abord à la figure 1 qui montre l'organisation générale d'un dispositif de fixateur externe à usage orthopédique selon l'invention.

Ce dispositif, propre à être utilisé pour la mise en oeuvre de la méthode dite d'ILIZAROV, comprend un premier anneau ou arceau 1, avantageusement constitué par la réunion à l'aide de rotules 2 et 3 de deux demi-arceaux, 1a et 1b respectivement, ainsi qu'un second anneau ou arceau 4, lui aussi avantageusement constitué par la réunion à l'aide de rotules 5 et 6 de deux demi-arceaux 4a et 4b, respectivement, les demi-arceaux 1a, 1b, comme les demi-arceaux 4a, 4b étant réalisés en un tube de section circulaire, présentant par exemple un diamètre de 12 mm.

Sur chacun des arceaux 1 et 4 sont montés des colliers articulés référencés 7, 8 et 9 lorsqu'ils sont associés à l'arceau 1 ou 7', 8' et 9' lorsqu'ils sont associés à l'arceau 4, et qui seront décrits plus en détail ci-après.

Les colliers articulés 7 et 8 sont prévus pour la fixation à l'arceau 1 de broches ou aiguilles 10 et 11 lesquelles, selon la méthode d'ILIZAROV, se croisent dans une structure osseuse 0 (montrée en traits mixtes) tandis que les colliers 7' et 8', associés à l'arceau 4, maintiennent sur ce dernier des aiguilles ou broches analogues 10' et 11', également prévues pour se croiser dans une structure osseuse, par exemple 0', opposée à la structure 0 par rapport à une fracture F lorsque le dispositif est destiné à être mis en oeuvre, par exemple, pour une ostéosynthèse, cette indication n'ayant bien entendu aucun caractère limitatif compte tenu du très grand nombre d'indications thérapeutiques possibles.

Dans la condition d'utilisation du dispositif les arceaux 1 et 4 sont reliés entre eux par trois entretoises tubulaires 12, de longueur réglable, dont chacune est constituée par une première partie 12a et une seconde partie 12b reliées entre elles par un système 13 à tige filetée et écrous moletés ou écrous huit pans 13a, 13b, 13c, ces deux derniers coopérant avec les parties 12a et 12b, respectivement, pour commander leur écartement et/ou leur rapprochement télescopique.

Pour la liaison des entretoises 12 aux arceaux 1 et 4, l'invention prévoit de faire application des colliers 9 et 9' auxquels sont associées, respectivement, des rotules 14 et 15 de structure analogue à celle référencées 2, 3 et 5, 6.

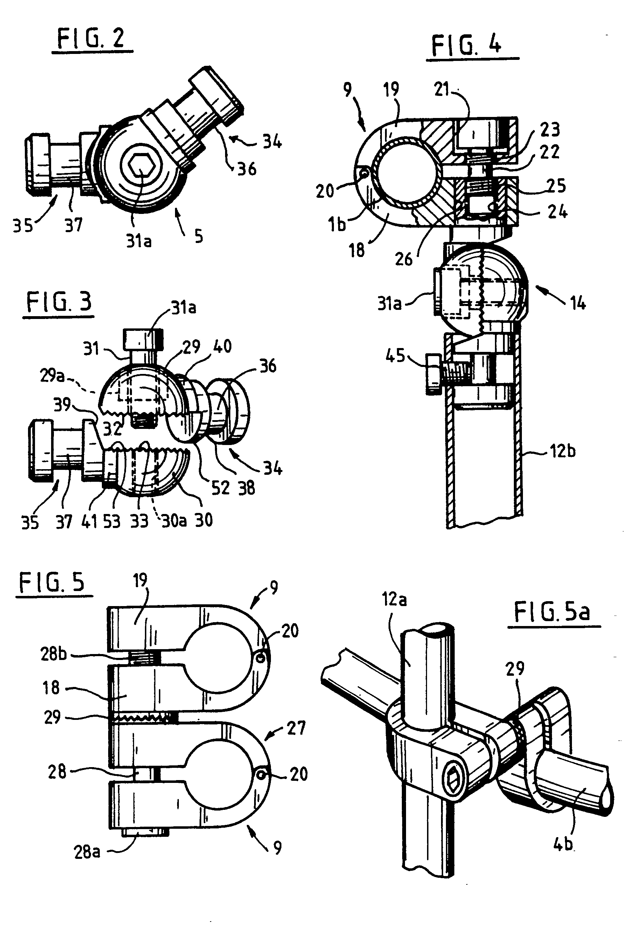

Comme montré sur les figures 2 et 3, chaque rotule 2, 3, 5 ou 6 se compose de deux parties à corps sensiblement hémisphérique 29, 30 qui sont assemblées au moyen d'un axe fileté 31 autour duquel elles sont mobiles l'une par rapport à l'autre, en rotation, l'une des demi-sphères, par exemple celle référencée 30, comportant un taraudage axial 30a tandis que l'autre demi-sphère 29 présente un logement 29a de réception de la tête creuse hexagonale 31a de l'axe fileté 31. Les deux faces planes diamétrales 32, 33, placés en regard l'une de l'autre, de chaque demi-sphère 29, 30 sont crantées, de sorte que ces deux demi-sphères peuvent être positionnées et bloquées l'une par rapport à l'autre en une multiplicité de positions angulaires stables avec leurs faces 32 et 33 au contact.

Chacune des demi-sphères 29, 30 porte un embout 34, 35 dirigé radialement et destiné à être inséré soit dans une entretoise tubulaire comme 12a ou 12b, soit dans l'une des extrémités ouvertes d'un demi-arceau formant les anneaux 1 ou 4, la fixation des rotules aux arceaux 1, 4 ou aux entretoises 12 étant assurée par une ou des vis de blocage 45, (figure 1), propre à coopérer avec une gorge périphérique 36, 37 de chacun des embouts 34, 35.

Les faces de ces derniers tournées vers les demi-sphères comportent chacune un dégagement annulaire 38, 39, qui permet la rotation des demi-sphères 29, 30 l'une par rapport à l'autre sans frottement, chaque demi-sphère comportant en outre un segment de collerette 40, 41, qui relie la demi-sphère à l'embout 34, 35, et qui sert en outre de butée de positionnement dans les tubes constitutifs des entretoises 12 ou des arceaux 1, 4. A la rotation sans frottement des deux parties constitutives de la rotule l'une par rapport à l'autre contribue la coopération d'un méplat 52, 53, ménagé sur chaque segment de collerette 40, 41, avec le dégagement 38, 39 de la face interne de l'embout 34, 35.

Les rotules 14 ou 15 de liaison des entretoises 12 aux arceaux 1 et 4 sont identiques à celles décrites ci-dessus, sous réserve de ce qu'un des embouts est simplement constitué par un canon 25 fileté intérieurement et destiné à coopérer avec un collier 9 ou 9'.

Celui-ci (figure 4), est composé de deux mâchoires 18, 19 articulées entre elles par une charnière 20, la mâchoire 19 présentant à son extrémité distante de l'articulation un évidement 21 de réception de la tête creuse hexagonale d'une tige en partie filetée 22 propre à coopérer avec un ou deux filets 23 du fond du logement 21, d'une part, et, d'autre part, avec le filetage 24 du canon 25 logé dans un forage 26 de la mâchoire 18.

Dans une autre forme de réalisation, (figure 5), les entretoises 12 — avantageusement constituées par des tubes rigides comportant des perforations (non représentées) — sont reliées aux arceaux 1 et 4 par une attache 27 rassemblant suivant un ensemble unitaire deux colliers tels que décrits ci-dessus. Chaque collier est mobile autour d'un axe 28 terminé à une extrémité par une tête creuse hexagonale 28a et à l'autre extrémité par une partie filetée 28b prévue pour coopérer avec un écrou imperdable, non représenté, logé dans la mâchoire du collier 9 la plus distante de celle prévue pour recevoir la tête d'actionnement 28a. Les deux mâchoires adjacentes des colliers 9 sont avantageusement crantées sur leurs faces en regard et dans leurs parties adjacentes aux débouchés des forages traversants de passage de l'axe 28, comme montré en 29, de sorte que les deux colliers constitutifs de l'attache peuvent être immobilisés angulairement de façon sûre, l'un par rapport à l'autre, ainsi que sur les entretoises et les arceaux, par actionnement de la tête 28a, le plus simplement à l'aide d'une clef Allen, la disposition étant alors celle montrée schématiquement sur la figure 5a.

Un tel agencement trouve particulièrement application dans les interventions chirurgicales qui mettent en oeuvre un allongement du segment osseux, tout en autorisant les mêmes réglages et ajustements angulaires que ceux permis par la liaison du type à collier et rotule illustrée sur la figure 4.

Pour l'application de la méthode d'ILIZAROV, le dispositif selon l'invention comprend en outre des moyens pour la mise en place, dans la ou les structures osseuses, des broches ou aiguilles 10, 11, 10' et 11'.

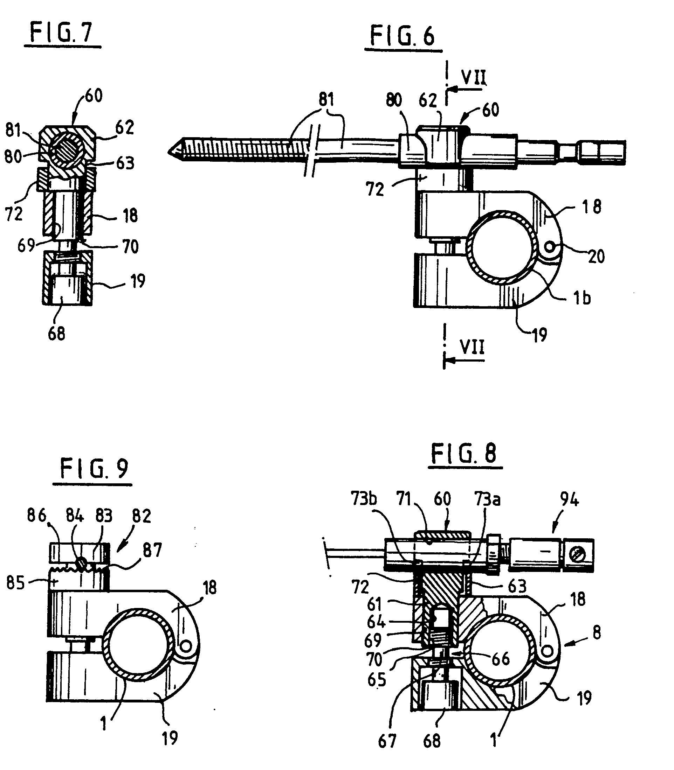

Lesdits moyens, montrés schématiquement sur la figure 1, comprennent des colliers articulés, tels que décrit ci-dessus, mais dans lesquels (figures 6 et 9), le forage 26 d'une des mâchoires (de façon plus précise de celle qui ne présente pas de logement de réception de la tête d'actionnement de la vis), reçoit une pièce 60 comportant une queue cylindrique 61 et une tête 62, également cylindrique, reliée à la queue 61 par un collet 63 de diamètre intermédiaire entre celui de la tête 62 et celui de la queue 61. Cette dernière présente un trou borgne taraudé axial 64 avec lequel est propre à coopérer l'extrémité filetée 65 d'une tige 66 à corps cylindrique 67 non fileté et qui se termine par une tête creuse hexagonale d'actionnement 68. La queue 61 de la pièce 60 est prévue pour être logée dans le forage traversant 69 de la mâchoire 18 dans laquelle elle est susceptible de tourner librement, mais dont elle ne peut s'échapper en raison de picots 70 formés sur la face d'extrémité de la queue 61 après que celle-ci ait été introduite dans le forage 69.

La tête 62 de la pièce 60 est percée d'un oeil 71, (figure 8), d'axe perpendiculaire à celui de la queue 61 et qui est d'un diamètre tel qu'il s'étende non seulement par une partie de la tête 62 mais également sur une partie du collet 63. Comme cela est bien visible sur les figures 6, 7 et 8, le collet 63 est entouré d'une bague 72 dont la largeur ou hauteur est choisie de manière telle que l'oeil 71 soit totalement dégagé lorsque la bague 72 est maintenue au contact de la mâchoire 18 du collier et que la pièce 60 en est éloignée autant que faire se peut, tandis que, comme cela est visible sur les figures 7 et 8, l'oeil 71 est en partie masqué lorsque, par actionnement de la tête 68 à l'aide d'une clef Allen, on provoque le rapprochement l'une de l'autre des mâchoires 18, 19. Ce mouvement, qui assure la fixation du collier articulé autour de l'arceau 1 ou 4, provoque, d'autre part, un déplacement relatif de la tête 62 de la pièce 60 par rapport à la bague 72 qui assure l'immobilisation de toute partie cylindrique préalablement introduite dans l'oeil 71 et dont le diamètre correspond au diamètre dudit oeil, ladite partie étant alors coincée entre la face interne de l'oeil et deux zones diamétralement opposées 73a, 73b de la tranche de la bague 72 opposée à celle reposant sur la mâchoire 18 (figure 8).

Dans une variante, non représentée, la tête 62 est à extrémité hexagonale, permettant ainsi un actionnement à l'aide d'une clé usuelle, pour sa mise en rotation.

Pour la mise en place du moyen de contention externe que constitue le dispositif suivant l'invention, on rapporte d'abord autour d'une structure osseuse, par exemple 0 sur la figure 1, un arceau 1 sur lequel sont fixés trois colliers comme les colliers 8. Dans l'oeil 71 de chacune des pièces 60 associée au collier 8 est placé un manchon 80 de guidage d'une fiche auto-taraudeuse 81 (figure 6), par exemple d'un diamètre de 4 mm alors que le diamètre de l'oeil 71 peut être de l'ordre de 8 mm.

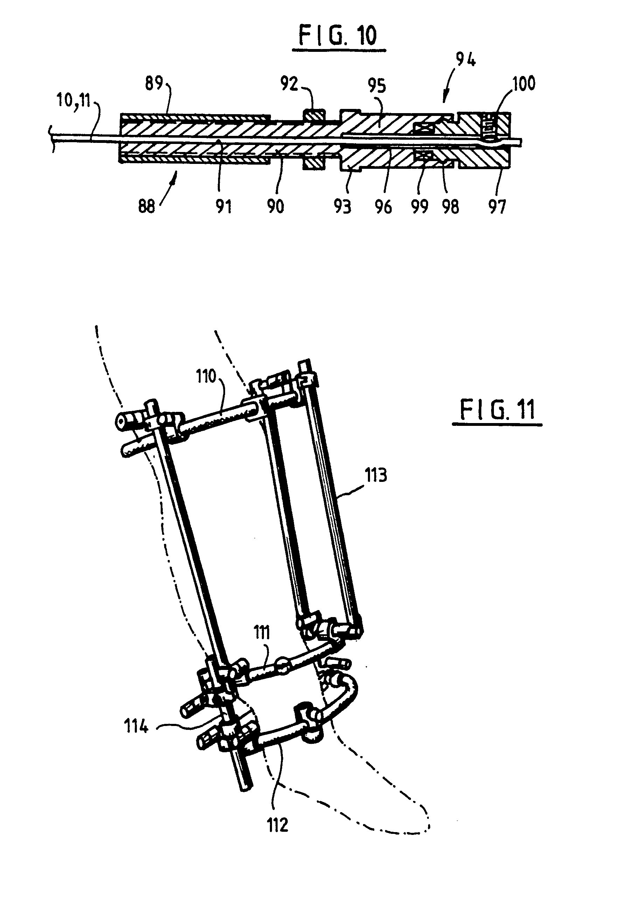

Les trois colliers étant disposés à 120° sur l'arceau 1, le chirurgien fait pénétrer les fiches 81 dans la structure osseuse, en ne perçant à chaque fois qu'une corticale puis met en place de façon précise l'arceau 1 par rapport à la structure osseuse 0, par manoeuvre des rotules 2 et 3 et des colliers 8. Si les mesures angulaires auxquelles il procède alors lui donnent satisfaction, il met en place les broches ou aiguilles 10, 11 — lesquelles ont généralement un diamètre de l'ordre de 1,8 à 2 mm — en faisant application des colliers 8 auxquels sont alors associés des moyens 82 de maintien desdites aiguilles ou broches (figure 9), et des organes servant à la fois au maintien et à la mise sous tension (mise en traction) desdites aiguilles ou broches, comme montré sur les figures 8 et 10. Les moyens 82 (figure 9), de maintien d'une extrémité d'une aiguille ou broche sont de structure analogue à celle décrite ci-dessus pour la pièce 60 sous réserve que la tête 83, correspondant à la tête 62, est percée d'un oeil 84 de plus petit diamètre tandis que l'anneau 85 — correspondant à l'anneau 72 — est muni sur sa face 86 en regard de la tête 83 de crans ou d'ergots 87.

Une extrémité d'une broche ou aiguille 10, 11 étant fixée dans les moyens 82, l'autre extrémité de ladite broche ou aiguille est reçue dans un organe 88 (figure 10), comportant un fourreau 89 à filetage interne prévu pour être logé dans l'oeil 71 d'une pièce 60. Avec le fourreau 89 coopère une tige filetée 90 percée d'un forage axial 91 de passage de la broche ou aiguille 10, 11 et avec laquelle est propre à coopérer, d'une part, un écrou de blocage 92 et, d'autre part, un écrou d'actionnement 93 d'un équipage mobile 94. Ce dernier comporte un manchon 95 à forage axial 96 de passage de la broche ou aiguille 10, 11, et qui est solidaire en translation d'un anneau 97 par l'intermédiaire d'ergots d'encliquetage 98 mais désolidarisé en rotation dudit anneau par l'intermédiaire de billes 99. L'anneau 97 est également percé axialement pour le passage de la broche ou aiguille 10, 11 et est muni de deux forages rayonnants 100 dans lesquels sont logés des vis à tête creuse hexagonale, l'actionnement de l'une d'entre elles déformant la broche ou aiguille 10, 11 — comme montré sur la figure 10 — pour immobiliser celle-ci par rapport à l'anneau.

Cette organisation de l'équipage 94 permet le mouvement de translation de l'ensemble, parallèlement à la direction de la broche ou aiguille, sans mettre celle-ci en rotation sur elle-même et, par conséquent, sans mouvement autre qu'un mouvement de translation dans la structure osseuse traversée par ladite broche ou aiguille.

Après que les broches ou aiguilles 10, 11 aient été mises en place, et cela avec une précision qui peut être de l'ordre de 5° d'angle, on retire les fiches auto-taraudantes 81, on met en traction les broches ou aiguilles 10, 11 à l'aide des organes 88, et l'on maintient cette traction, qui peut être de l'ordre de 100 kg, en amenant l'écrou 92 au contact du fourreau 89.

On procède de la même manière pour la mise en place de l'anneau 4 et des aiguilles qui lui sont associées puis l'on réunit les deux arceaux 1 et 4 au moyen des entretoises 12.

La présence des rotules comme 2, 3, 5, 6 sur les arceaux et des rotules 14 et 15 sur les entretoises, de même que l'emploi d'attaches 27 à deux colliers articulés procure un nombre élevé de degrés de liberté offrant au chirurgien des possibilités multiples d'applications.

En outre, l'organisation du dispositif tel que décrite ci-dessus autorise, au cours de l'évolution de la consolidation d'une fracture, des déplacements des deux arceaux l'un par rapport à l'autre, ce qui est d'importance primordiale pour l'obtention de résultats thérapeutiques satisfaisants, en particulier dans les interventions où l'on constate, après un certain délai, un défaut de réduction de fracture.

Le dispositif selon l'invention n'est pas, bien entendu, limité à ce type d'intervention.

Ainsi, et comme montré sur la figure 11 à titre d'exemple, un dispositif selon l'invention faisant application de trois arceaux 110, 111, 112 auxquels sont associés respectivement, trois jeux de broches ou aiguilles 10, 11 et deux jeux d'entretoises, respectivement 113 entre les arceaux 110 et 111, et 114 entre les arceaux 111 et 112, peut trouver application pour le traitement de fracture basse de la cheville, ou encore de fractures dites bi-focales.

Quelque soit le type d'intervention, l'organisation d'un dispositif selon l'invention peut être modifié et réglée jusqu'à guérison complète ce qui contribue encore à l'intérêt important du dispositif.

**Revendications**

1. Dispositive de fixateur externe à usage orthopédique propre à être utilisé pour la mise en oeuvre de la méthode dite d'ILIZAROV, comprenant au moins deux anneaux (1, 4) pour la fixation d'aiguilles croisées (10, 11, 10′, 11′), des entretoises (12) étant propres à être reliées auxdits anneaux (1, 4) et comportant des moyens (13) de réglage de leur longueur pour la mise en traction ou en compression d'un os et/ou fragments d'os (0, 0′) auxquels sont reliés lesdits anneaux par lesdites aiguilles (10, 11, 10′, 11′), des moyens (94) étant en outre prévus pour la mise en traction desdites aiguilles (10, 11, 10′, 11′), caractérisé en ce que les anneaux (1, 4) sont formés d'un tube de section circulaire sur lequel sont fixés et immobilisés en position des colliers articulés (7, 8, 9 — 7′, 8′, 9′) reliés respectivement, après positionnement et blocage en position angulaire, aux aiguilles (10, 11, 10′, 11′) et aux entretoises (12), les colliers (7, 7′, 8, 8′) reliés aux aiguilles (10, 11, 10′, 11′) recevant de façon orientable et amovible des organes (60) comportant des moyens (94) de mise en traction réglable desdites aiguilles (10, 11 — 10′—11′) tandis que les colliers (9, 9′) reliés aux entretoises (12) comportent des moyens d'assemblage en articulation (14, 29, 30).

2. Dispositif selon la revendication 1, caractérisé en ce que les anneaux sont formés de deux demi-arceaux (1a, 1b 4a, 4b) reliés par des articulations du type à rotule crantée (2, 3, 5, 6).

3. Dispositif selon l'une quelconque des revendications 1 ou 2, caractérisé en ce que les organes (60, 94) de fixation et de mise en traction réglable des broches ou aiguilles (10, 10', 11, 11') comprennent un fourreau taraudé (89) propre à être introduit dans l'oeil (71) d'une pièce (62) coopérant avec un collier articulé (8) et un équipage (94) comportant un anneau (97) de fixation de l'extrémité de la broche ou aiguille (10, 11) auquel est associé en translation un manchon (95) solidaire d'un écrou (93) prolongé par une tige filetée (90), qui coopère avec le fourreau (89), ledit manchon (95) étant monté à rotation par rapport à l'anneau (97) au moyen de billes (99) ou analogues.

4. Dispositif selon la revendication 3, caractérisé par le fait qu'à la place de l'équipage (94) peut être préalablement monté un dispositif de forage (80, 81).

**Patentansprüche**

1. Orthopädische Vorrichtung zur äusseren Fixierung von Knochen und verwendbar bei Anwendung der Ilizarov Methode, welche wenigstens zwei Ringe (1, 4) zur Befestigung von gekreuzten Nadeln (10, 11, 10', 11') mit Streben (12), die zur Verbindung mit diesen Ringen (1, 4) geeignet sind, und Mittel (13) zur Einstellung ihrer Länge für den Zug oder das Zusammendrücken von einem Knochen und/oder Knochenfragmenten (0, 0') mit denen die Ringe durch die Nadeln (10, 11; 10', 11') verbunden sind, enthält, wobei weitere Mittel (94) für den Zug dieser Nadeln (10, 11; 10', 11') vorgesehen sind, dadurch gekennzeichnet, dass die Ringe (1, 4) aus einem Rohr mit kreisförmigem Schnitt bestehen auf welchem Gelenkklemmen (7, 8, 9, — 7', 8', 9') befestigt und in Position festgehalten sind, die jeweils, nach Positionierung und Verriegelung in Winkelstellung mit den Nadeln (10, 11; 10', 11') und den Streben (12) verbunden sind, wobei die mit den Nadeln (10, 11; 10', 11') verbundenen Klemmen (7, 7'; 8, 8') auf orientierbare und abnehmbare Weise Glieder (60) aufnehmen, welche Mittel (94) für regulierbaren Zug der Nadeln (10, 11; 10', 11') aufweisen, während die mit den Streben (12) verbundenen Klemmen (9, 9') Gelenkverbindungsmittel (14, 29, 30) aufweisen.

2. Vorrichtung nach Anspruch 1, dadurch gekennzeichnet, dass die Ringe aus zwei Halbringen (1a, 1b, 4a, 4b) bestehen, welche durch Gelenke des gerasteten Kugelgelenk-Typs (2, 3, 5, 6) verbunden sind.

3. Vorrichtung nach einem der Ansprüche 1 oder 2, dadurch gekennzeichnet, dass die Glieder (60, 94) zur Fixierung und regulierbarem Zug der Stifte oder Nadeln (10, 10'; 11, 11') eine Innengewindehülse (89) zur Einführung in das Auge (71) eines Teils (62) aufweisen, welches mit einer Gelenkklemme (8) und einem beweglichen Organ (94) zusammenarbeitet, welches einen Ring (97) zur Befestigung des Stift- oder Nadelendes (10, 11) aufweist, dem in Längsverschiebung eine an einer Schraubenmutter (93) befestigte und durch eine Schraubenspindel (90) verlängerte Hülse (95) angeordnet ist, welche mit der Innengewindehülse (89) zusammenarbeitet, wobei die Hülse (95) mittels Kugeln oder ähnlichem drehend zu dem Ring (97) gelagert ist.

4. Vorrichtung nach Anspruch 3, dadurch gekennzeichnet, dass anstelle des beweglichen Organs (94) eine Bohrvorrichtung (80, 81) vormontierbar ist.

**Claims**

1. External fixing device for orthopaedic use suitable for use in carrying out the method known as the Ilizarov method, comprising at least two annular members (1, 4) for fastening crossed elongated pin members (10, 11, 10', 11'), bracing members (12) adapted to be joined to said annular members (1, 4) and including means (13) for adjusting the length thereof for putting a bone and/or bone fragments (0, 0') under traction or compression, said bone or bone fragments being joined to said annular members by said elongated pin members (10, 11, 10', 11'), means (94) being further provided for putting said elongated pin members (10, 11, 10', 11') under traction, characterized in that the annular members (1, 4) are formed by a circular cross-section tube having pivoted clamps (7, 8, 9 — 7', 8', 9') rigidly and non-movably affixed thereto said clamps being respectively joined, after positioning and locking at their angular position, to said elongated pin members (10, 11, 10', 11'), and to said bracing members (12), the clamps (7, 7', 8, 8') joined to the elongated pin members (10, 11, 10', 11') receiving, in an orientable and removable manner, members (60) having means for putting said elongated pin members (10, 11, 10', 11') under adjustable traction while the clamps (9, 9') joined to the bracing members (12) include pivoted assembling means (14, 29, 30).

2. Device according to claim 1 characterized in that the annular members are made up by two semi-circular parts (1a, 1b, 4a, 4b) linked by serrated ball-type swivel joints (2, 3, 5, 6).

3. Device according to either one of claims 1 or 2 characterized in that the members (60, 94) for fixing and putting said elongated pin members (10, 11, 10', 11') under adjustable traction include an internally threaded member (89) adapted to be introduced into the passage (71) of a part (62) cooperating with a pivoted clamp (8), and a rotatable element (94) including a fixing ring (97) for the end of an elongated pin member (10, 11) said ring being associated in translatory motion with a sleeve (95) integral with a screw nut member (93) extended by a threaded rod (90), said rod cooperating with said internally threaded member (89), said sleeve (95) being rotatably mounted with respect to the fixing ring (97) by means of ball bearings (99) or similar.

4. Device according to claim 3, characterized in that a drilling device (80, 81) can be pre-mounted as a replacement for said rotatable element (94).

FIG.1

FIG. 2

FIG. 4

FIG. 3

FIG. 5

FIG. 5a

FIG. 7

FIG. 6

FIG. 9

FIG. 8

FIG. 10

FIG. 11